# FASCICULE DE BREVET EUROPEEN

(11) **EP 4 153 029 B1**
(45) Date de publication et mention de la délivrance du brevet: **09.07.2025**
(21) Numéro de dépôt: 21732471.4
(22) Date de dépôt: 18.05.2021
(51) Int. Cl.: A61B 5/00, A44C 5/00

(54) **SYSTÈME DE SURVEILLANCE CORPORELLE AJUSTABLE**
EINSTELLBARES KÖRPERÜBERWACHUNGSSYSTEM
ADJUSTABLE BODY MONITORING SYSTEM

(30) Priorité: 20.05.2020 FR 2005349
(43) Date de publication de la demande: 29.03.2023
(73) Titulaire: WIZP AS, 3183 Horten (NO)
(72) Inventeur: PIERART, Luc, 94800 VILLEJUIF (FR)
(74) Mandataire: Regimbeau
(86) Numéro de dépôt international: PCT/FR2021/050859
(87) Numéro de publication internationale: WO 2021/234266

(56) Documents cités:
- WO-A1-2019/181266
- WO-A1-2020/025820
- US-A1- 2007 191 696

## Description

### DOMAINE DE L'INVENTION

La présente convention concerne un dispositif de surveillance corporelle portable et ajustable, particulièrement adapté pour la détection d'un analyte corporel.

### ETAT DE LA TECHNIQUE

Un dispositif de surveillance corporelle ajustable comprend de manière connue un bracelet propre à entourer un membre d'un utilisateur, par exemple un poignet. L'ajustement d'un tel dispositif est réalisé au gré des sensations de l'utilisateur, ce qui peut entraîner un biais dans les mesures de grandeurs physiologiques mises en œuvre par le dispositif, telles que les mesures de glucose ou de lactate. Il n'est pas possible pour l'utilisateur de reconnaître ou d'identifier ces variations avec les dispositifs de mesure connus, ni d'ajuster à un certain niveau de serrage de façon certaine et répétée le dispositif autour d'un membre de l'utilisateur. Des systèmes de surveillance corporelle ajustables sont connus de WO-2019/181266-A1, WO-2020/025820-A1, et US-2007/191696A1.

### EXPOSE DE L'INVENTION

Un but de l'invention est de proposer une solution pour contrôler avec précision l'ajustement d'un dispositif de surveillance corporelle sur un utilisateur.

Ce but est atteint dans le cadre de la présente invention grâce à un système de surveillance corporelle ajustable selon la revendication 1.

Le système peut avantageusement comprendre les caractéristiques suivantes, prises individuellement ou en l'une quelconque de leurs combinaisons techniquement possibles :
- la première lanière comprend une deuxième face, opposée à la première face, chaque encoche formant un ajour traversant la première lanière, la deuxième face comprenant une pluralité de marqueurs, chaque marqueur étant formé par un relief ou une variation d'une couleur entre la surface du marqueur et le reste de la deuxième face, par rapport au reste de la deuxième face, une partie de chaque marqueur étant agencée à une distance inférieure ou égale au pas p d'une encoche dite associée audit marqueur, préférentiellement à une distance inférieure à la moitié du pas p de l'encoche associée, de sorte à former un deuxième réseau unidimensionnel de marqueurs, deux marqueurs adjacents du deuxième réseau unidimensionnel de marqueurs présentant des formes différentes et/ou des couleurs réfléchies par la deuxième face différentes, et/ou des orientations principales différentes dans le référentiel spatial de la première lanière,
- une encoche est associée à un marqueur associé de la première face et à un marqueur associé de la deuxième face, lesdits marqueurs associés présentant des formes identiques et/ou des couleurs réfléchies par la première face et par la deuxième face identiques, et/ou des orientations principales identiques dans le référentiel de la première lanière,
- le premier réseau unidimensionnel de marqueurs et/ou le deuxième réseau unidimensionnel de marqueurs présente au moins deux marqueurs présentant des formes différentes et/ou des couleurs réfléchies par la première face et/ou par la deuxième face, et/ou des orientations principales différentes dans le référentiel de la première lanière, les marqueurs étant agencés de manière périodique dans le premier réseau unidimensionnel de marqueurs et/ou le deuxième réseau unidimensionnel,
- le corps principal comprend des microaiguilles configurées pour mesurer une grandeur physiologique de l'utilisateur.

### DESCRIPTION DES FIGURES

D'autres caractéristiques, buts et avantages de l'invention ressortiront de la description qui suit, qui est purement illustrative et non limitative, et qui doit être lue en regard des dessins annexés sur lesquels :
- la figure 1 illustre schématiquement un dispositif selon un mode de réalisation de l'invention ;
- les figures 2 et 3 illustrent respectivement deux manières d'attacher un dispositif de l'invention au poignet d'un utilisateur ;
- les figures 4a, 4b, 4c, 4d et 4e illustrent plusieurs manières de prévoir les encoches et les marqueurs dans le dispositif de l'invention.

Sur l'ensemble des figures, les éléments similaires portent des références identiques.

### DEFINITIONS

On entend par deux « couleurs différentes réfléchies par une face » des couleurs présentant chacune une longueur d'onde, la différence entre les deux longueurs d'onde étant d'au moins 50 nm, préférentiellement 100 nm. Les couleurs sont réfléchies par la surface d'une lanière dans l'invention (en opposition à une transmission).

On entend par « réseau unidimensionnel » un arrangement systématique, préférentiellement périodique, d'éléments suivant une direction par rapport à un élément comprenant le réseau unidimensionnel.

On entend par « relief » une partie d'une surface surélevée et/ou enfoncée par rapport au reste de la surface, la valeur de la surélévation ou de l'enfoncement étant préférentiellement comprise entre 10 µm et 5 mm.

### DESCRIPTION DETAILLEE DE L'INVENTION

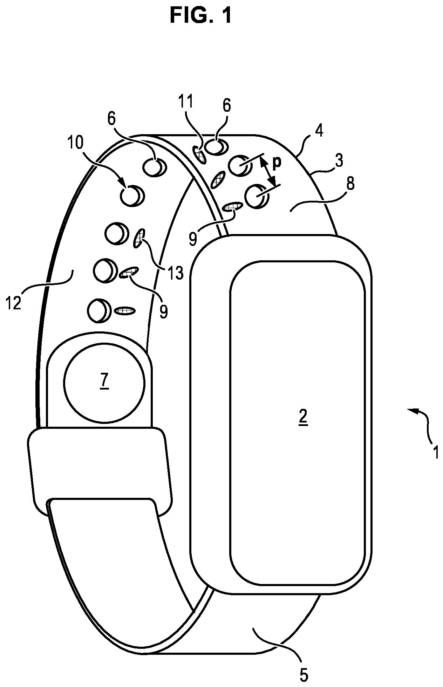

En référence à la figure 1, le système 1 de surveillance corporelle ajustable comprenant un corps principal 2 adapté pour mesurer une grandeur physiologique d'un utilisateur. Le corps principal 2 peut comprendre une unité de commande, l'unité de commande comprenant un microprocesseur et une mémoire. Le corps principal 2 peut comprendre préférentiellement une ou plusieurs microaiguilles, au moins une microaiguille étant configurée pour mesurer un analyte corporel. Les microaiguilles peuvent être recouvertes, au moins en partie, par une enzyme propre à réagir spécifiquement avec un analyte corporel. Une microaiguille peut également comprendre une électrode, permettant de relier la microaiguille à l'unité de commande. Le corps principal peut également comprendre une unité d'affichage, par exemple un écran, permettant à l'utilisateur de lire une valeur représentative de la détection de l'analyste corporelle.

Le système comprend un bracelet configuré pour entourer un membre de l'utilisateur. Le bracelet comprend une première lanière 4 montée sur le corps principal 2 et une deuxième lanière 5 montée sur le corps principal 2. Chacune des lanières est montée sur le corps principal à une extrémité de la lanière. Une lanière peut être montée fixe, ou par une liaison permettant une rotation entre le corps principal 2 et la lanière.

La première lanière 4 comprend un réseau unidimensionnel d'encoches 10. Une encoche 6 peut-être un renfoncement dans la lanière ou bien un ajour traversant la lanière. Le réseau unidimensionnel d'encoches 10 présente un pas *p.* Le pas *p* est défini comme la distance suivant la lanière entre deux centres d'encoches 6. Le pas *p* est préférentiellement égal entre chacune des encoches 6 adjacentes par rapport à la première lanière 4.

La deuxième lanière 5 comprend des moyens d'attache 7 configurés pour être fixés de manière amovible dans l'une des encoches 6. Les moyens d'attache 7 peuvent être mis en œuvre par une saillie de forme complémentaire à l'encoche 6, ou traversant l'encoche 6, par un fermoir à boucles de type ardillon, par un fermoir à boucle déployante simple ou double, par un fermoir à clip, ou par tous autres moyens d'attache connu de la personne du métier.

La première lanière 4 comprend une première face 8. La première face 8 comprend une pluralité de marqueurs 9. Chaque marqueur 9 est formé par un relief et/ou une variation d'une couleur réfléchie par la première face 8 sur la surface du marqueur 9 par rapport au reste de la première face 8. Une partie de chaque marqueur 9 est agencée à une distance inférieure ou égale au pas *p* d'une encoche 6. Cette encoche 6 est dite « associée » audit marqueur 9 et *vice versa.* Une encoche 10 peut être associée à un marqueur 9 unique, ou ne pas être associé à un marqueur, et un marqueur est associé à une encoche unique. Une partie du marqueur 9 associé à une encoche 6 est préférentiellement agencée à une distance inférieure à la moitié du pas *p* de l'encoche 6 associée, de sorte à former un premier réseau unidimensionnel 11 de marqueurs 9. Deux marqueurs 9 adjacents du premier réseau unidimensionnel 11 de marqueurs 9 présentent des formes différentes et/ou des couleurs réfléchies par la première face 8 différentes, et/ou des orientations principales identiques dans le référentiel de la première lanière 4. Ainsi, l'agencement des différents marqueurs permet à un utilisateur de contrôler avec précision le serrage ou le desserrage du bracelet entourant le membre de l'utilisateur. On entend par « orientation principale » le sens d'un axe du marqueur porté par la plus grande dimension du marqueur.

La première lanière 4 comprend préférentiellement une deuxième face 12, opposée à la première face 8. Selon le mode de réalisation de l'invention, la première face 8 peut-être la face propre à être utilisé en regard du membre de l'utilisateur, ou la face propre à être visible une fois le bracelet ajusté aux membres de l'utilisateur. Chaque encoche 6 forme, dans ce mode de réalisation de l'invention, un ajour traversant la première lanière 4. La deuxième face 12 comprenant une pluralité de marqueurs 9, chaque marqueur 9 étant formé par un relief ou une variation d'une couleur réfléchie par la deuxième face 12 sur la surface du marqueur 9, par rapport au reste de la deuxième face 12. Une partie de chaque marqueur 9 est agencée à une distance inférieure ou égale au pas *p* d'une encoche 6 associée audit marqueur 9, et préférentiellement à une distance inférieure à la moitié du pas *p* de l'encoche 6 associée, de sorte à former un deuxième réseau unidimensionnel 13 de marqueurs. Deux marqueurs 9 adjacents du deuxième réseau unidimensionnel 13 de marqueurs présentent des formes différentes et/ou des couleurs réfléchies par la deuxième face 8 différentes, et/ou des orientations principales identiques dans le référentiel de la première lanière 4. En effet, le serrage du dispositif 1 peut être contrôlé par l'utilisateur selon l'évolution des marqueurs 9 de la première face 8, tandis que le desserrage du dispositif 1 peut être contrôlé par l'utilisateur selon l'évolution des marqueurs de la deuxième face 12 opposée à la première face 8.

Une encoche 6 peut être préférentiellement associée à la fois à un marqueur 9 associé sur la première face 8 et à la fois à un marqueur 9 associé sur la deuxième face 12. Lesdits marqueurs 9 associés présentent alors des formes identiques et/ou des couleurs réfléchies par la première face 8 et par la deuxième face 12 identiques, et/ou des orientations principales identiques dans le référentiel de la première lanière 4.

Le premier réseau unidimensionnel 11 de marqueurs et/ou le deuxième réseau unidimensionnel 13 de marqueurs présente(nt) au moins deux marqueurs 9, les marqueurs 9 présentant des formes différentes et/ou des couleurs différentes réfléchies par la première face 8 et/ou par la deuxième face 12, et/ou des orientations principales différentes dans le référentiel de la première lanière 4, les marqueurs 9 étant agencés de manière périodique dans le premier réseau unidimensionnel 11 de marqueurs et/ou le deuxième réseau unidimensionnel 13. Ainsi, il est possible de simplifier la fabrication du bracelet en limitant le nombre de marqueurs différents tout en permettant le contrôle du serrage et/ou du desserrage la longueur de la première lanière 4.

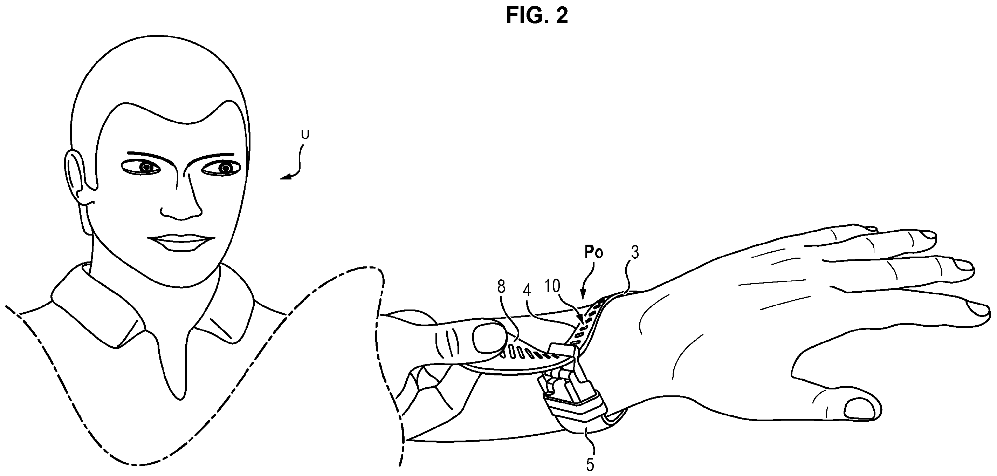

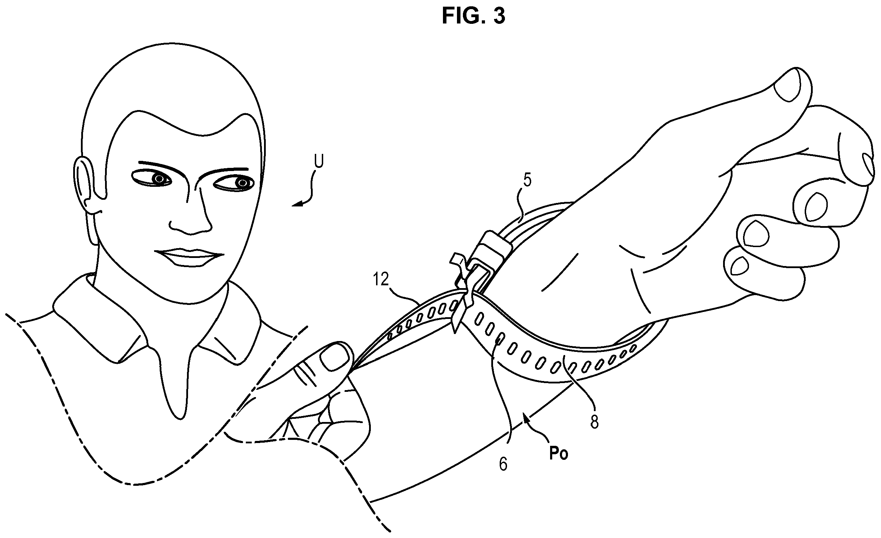

Selon un mode de réalisation préféré, les encoches et les marqueurs associés sur chacune des faces sont identiques. Ceci est particulièrement avantageux car cela rend visible par le porteur les encoches et les marqueurs quel que soit la manière de mettre la montre autour du poignet. En effet, le porteur doit pouvoir mettre sa montre qu'il soit gaucher ou droitier, avec donc la lanière orientée différemment. Ceci est illustré sur les figures 2 et 3.

Sur les figures 2 et 3 la lanière 4 du bracelet 3 est présentée de deux manières différentes à l'utilisateur U. On constate que l'utilisateur U peut visualiser selon la manière dont est installée le système à son poignet PO soit la première face 8, soit la deuxième face 12 et donc les marqueurs et les encoches de la face qu'ils lui sont présentés afin d'installer correctement le système à son poignet, c'est-à-dire insérer les moyens d'attache 7 (une boucle par exemple) dans une encoche 6, 10. En particulier, sur la figure 2, c'est la première face 8 qui est présentée à l'utilisateur U tandis que sur la figure 3 c'est la deuxième face qui est présentée à l'utilisateur U. Outre le fait que le bracelet peut être facilement installé indifféremment sur le bras gauche ou le bras droit il permet de changer le bracelet sans se préoccuper d'un quelconque sens d'installation. N'importe comment, l'utilisateur pourra installer le dispositif à son poignet et aura toujours en visuel les marqueurs et les encoches.

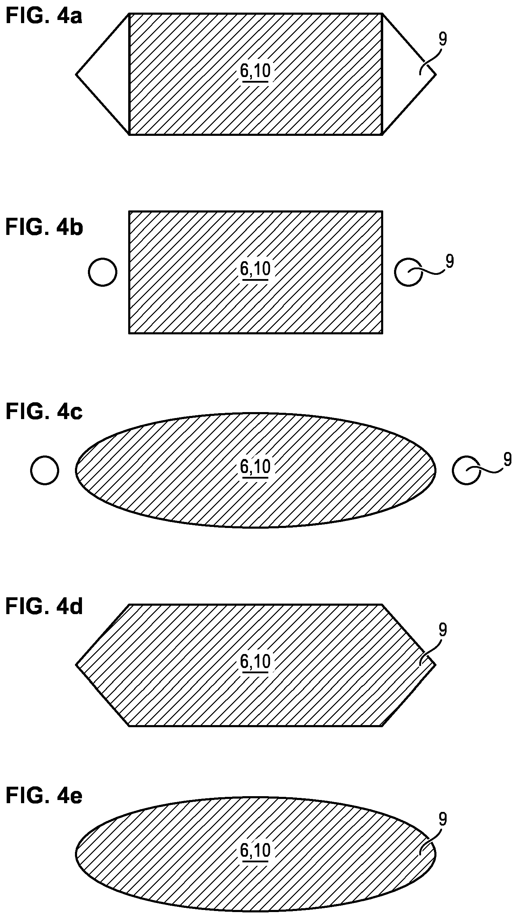

Selon un mode de réalisation, les premier et deuxième réseau de marqueurs 9 sont identiques, et chaque marqueur 9 est accolé à l'encoche 6, 10 à laquelle il est associé, chaque marqueur 9 associé à chaque encoche consécutive étant différent. Ceci est illustré sur les figures 4a, 4b et 4c. sur la figure 4a, l'encoche 6, 10 est rectangulaire et le marqueur associé est triangulaire et est formé par relief (la partie hachurée représente une ouverture tandis que la partie non hachurée représente un relief). Sur la figure 4b, l'encoche 6, 10 est est rectangulaire et le marqueur associé est un cercle (la partie hachurée représente une ouverture tandis que la partie non hachurée représente un relief). Sur la figure 4c, l'encoche 6, 10 est ovoïde est le marqueur associé est un cercle (la partie hachurée représente une ouverture tandis que la partie non hachurée représente un relief). On note que sur les figures 4a, 4b et 4c des marqueurs sont situés de part et d'autre de l'encoche 6, 10 mais on peut envisager un marqueur 9 d'un seul côté de l'encoche 6, 10.

Selon un mode de réalisation les premier et deuxième réseau de marqueurs sont identiques, et le marqueur est constitué par une forme donnée à l'encoche de telle sorte qu'au moins chaque encoche consécutive présente une forme différente. Ceci est illustré sur les figures 4d et 4e où c'est la forme de l'encoche en tant que telle qui permet de distinguer chaque adjacente. L'homme du métier comprend que l'on peut donner n'importe quelle forme à une encoche pourvu que deux encoches adjacentes soient différentes.

En outre, ceci permet au porteur d'ajuster correctement le bracelet simplement.

## Revendications

1. Système (1) de surveillance corporelle ajustable comprenant :
- un corps principal (2) adapté pour mesurer une grandeur physiologique d'un utilisateur,
- un bracelet (3) configuré pour entourer un membre de l'utilisateur, le bracelet comprenant une première lanière (4) montée sur le corps principal (2) et une deuxième lanière (5) montée sur le corps principal (2), la première lanière (4) comprenant un réseau unidimensionnel d'encoches (10), le réseau unidimensionnel d'encoches (10) présentant un pas p, la deuxième lanière (5) comprenant des moyens d'attache (7) configuré pour être fixé de manière amovible dans l'une des encoches (6), chaque encoche formant un ajour traversant la première lanière, où :
- une première face (8) de la première lanière (4) comprend une pluralité de marqueurs (9), chaque marqueur (9) étant formé par un relief ou une variation d'une couleur entre la surface du marqueur (9) et le reste de la première face (8), une partie de chaque marqueur (9) étant agencée à une distance inférieure ou égale au pas *p* d'une encoche (6) associée audit marqueur (9), préférentiellement à une distance inférieure à la moitié du pas p de l'encoche (6) associée, de sorte à former un premier réseau unidimensionnel (11) de marqueurs, deux marqueurs (9) adjacents du premier réseau unidimensionnel (11) de marqueurs présentant des formes différentes et/ou des couleurs différentes réfléchies par la première face (8), et/ou des orientations principales différentes dans le référentiel spatial de la première lanière (4),
- une deuxième face (12) de la première lanière (4) opposée à la première face (8), comprenant une pluralité de marqueurs (9), chaque marqueur (9) étant formé par un relief ou une variation d'une couleur entre la surface du marqueur (9) et le reste de la deuxième face (8), une partie de chaque marqueur (9) étant agencée à une distance inférieure ou égale au pas *p* d'une encoche (6) associée audit marqueur (9), préférentiellement à une distance inférieure à la moitié du pas *p* de l'encoche (6) associée, de sorte à former un deuxième réseau unidimensionnel (13) de marqueurs, deux marqueurs (9) adjacents du deuxième réseau unidimensionnel (13) de marqueurs présentant des formes différentes et/ou des couleurs différentes réfléchies par la deuxième face (8), et/ou des orientations principales différentes dans le référentiel spatial de la première lanière (4).

2. Système selon la revendication 1, dans lequel une encoche (6) est associée à un marqueur (9) associé sur la première face et à un marqueur (9) associé sur la deuxième face, lesdits marqueurs associés présentant des formes identiques et/ou des couleurs réfléchies par la première face (8) et par la deuxième face (12) identiques et/ou des orientations principales identiques dans le référentiel de la première lanière (4).

3. Système selon l'une des revendications précédentes, dans lequel les premier et deuxième réseau de marqueurs sont identiques, chaque marqueur est accolé à l'encoche à laquelle il est associé, chaque marqueur associé à chaque encoche consécutive étant différent.

4. Système selon l'une des revendications précédentes, dans lequel les premier et deuxième réseau de marqueurs sont identiques, le marqueur est constitué par une forme donnée à l'encoche de telle sorte qu'au moins chaque encoche consécutive présente une forme différente.

5. Système (1) selon la revendication précédentes, dans lequel le premier réseau unidimensionnel (11) de marqueurs et/ou le deuxième réseau unidimensionnel (13) de marqueurs présente au moins deux marqueurs (9) présentant des formes différentes et/ou des couleurs différentes réfléchies par la première face (8) et/ou par la deuxième face (12), et/ou des orientations principales différentes dans le référentiel de la première lanière (4), les marqueurs (9) étant agencés de manière périodique dans le premier réseau unidimensionnel (11) de marqueurs et/ou le deuxième réseau unidimensionnel (13).

6. Système (1) selon l'une des revendications précédentes, dans lequel le corps principal (2) comprend des microaiguilles configurées pour mesurer une grandeur physiologique de l'utilisateur.

## Patentansprüche

1. Einstellbares Körperüberwachungssystem (1), umfassend:
- einen Hauptkörper (2), der zur Messung einer physiologischen Größe eines Benutzers geeignet ist,
- ein Armband (3), das ausgelegt ist, um eine Gliedmaße des Benutzers zu umgeben, wobei das Armband ein erstes Band (4), das am Hauptkörper (2) angebracht ist, und ein zweites Band (5), das am Hauptkörper (2) angebracht ist, umfasst, wobei das erste Band (4) ein eindimensionales Kerben-Netzwerk (10) umfasst, wobei das eindimensionale Kerben-Netzwerk (10) einen Abstand *p* aufweist, wobei das zweite Band (5) Befestigungsmittel (7) umfasst, die ausgelegt sind, um lösbar in einer der Kerben (6) befestigt zu sein, wobei jede Kerbe einen Durchbruch bildet, der das erste Band durchquert, wobei:
- eine erste Seite (8) des ersten Bandes (4) eine Vielzahl von Markierungen (9) umfasst, wobei jede Markierung (9) durch ein Relief oder eine Änderung einer Farbe zwischen der Oberfläche der Markierung (9) und dem Rest der ersten Seite (8) gebildet wird, wobei ein Teil jeder Markierung (9) in einem Abstand eingerichtet ist, der kleiner oder gleich dem Abstand *p* einer der Markierung (9) zugeordneten Kerbe (6) ist, vorzugsweise in einem Abstand, der kleiner ist als die Hälfte des Abstands *p* der zugeordneten Kerbe (6), so dass ein erstes eindimensionales Markierungs-Netzwerk (11) gebildet wird, wobei zwei benachbarte Markierungen (9) des ersten eindimensionalen Markierungs-Netzwerks (11) unterschiedliche Formen und/oder unterschiedliche Farben aufweisen, die von der ersten Seite (8) reflektiert werden, und/oder unterschiedliche Hauptausrichtungen im räumlichen Bezugssystem des ersten Bandes (4),
- eine zweite Seite (12) des ersten Bandes (4), die der ersten Seite (8) gegenüberliegt, die eine Vielzahl von Markierungen (9) umfasst, wobei jede Markierung (9) durch ein Relief oder eine Änderung einer Farbe zwischen der Oberfläche der Markierung (9) und dem Rest der zweiten Seite (8) gebildet wird, wobei ein Teil jeder Markierung (9) in einem Abstand eingerichtet ist, der kleiner oder gleich dem Abstand p einer der Markierung (9) zugeordneten Kerbe (6) ist, vorzugsweise in einem Abstand, der kleiner ist als die Hälfte des Abstands *p* der zugeordneten Kerbe (6), um ein zweites eindimensionales Markierungs-Netzwerk (13) zu bilden, wobei zwei benachbarte Markierungen (9) des zweiten eindimensionalen Markierungs-Netzwerks (13) unterschiedliche Formen und/oder von der zweiten Seite (8) reflektierte unterschiedliche Farben, und/oder unterschiedliche Hauptausrichtungen im räumlichen Bezugssystem des ersten Bandes (4) aufweisen.

2. System nach Anspruch 1, wobei eine Kerbe (6) einer Markierung (9) zugeordnet ist, die auf der ersten Seite zugeordnet ist, und einer Markierung (9), die auf der zweiten Seite zugeordnet ist, wobei die zugeordneten Markierungen identische Formen und/oder von der ersten Seite (8) und der zweiten Seite (12) reflektierte identische Farben und/oder identische Hauptausrichtungen im Bezugssystem des ersten Bandes (4) aufweisen.

3. System nach einem der vorhergehenden Ansprüche, wobei das erste und das zweite Markierungs-Netzwerk identisch sind, wobei jede Markierung an die Kerbe angrenzt, der sie zugeordnet ist, wobei jede Markierung, die jeder aufeinanderfolgenden Kerbe zugeordnet ist, unterschiedlich ist.

4. System nach einem der vorhergehenden Ansprüche, wobei das erste und das zweite Markierungs-Netzwerk identisch sind, die Markierung aus einer der Kerbe gegebenen Form derart besteht, dass mindestens jede aufeinanderfolgende Kerbe eine unterschiedliche Form aufweist.

5. System (1) nach vorhergehendem Anspruch, wobei das erste eindimensionale Markierungs-Netzwerk (11) und/oder das zweite eindimensionale Markierungs-Netzwerk (13) mindestens zwei Markierungen (9) aufweist, die unterschiedliche Formen und/oder von der ersten Seite (8) und/oder von der zweiten Seite (12) reflektierte unterschiedliche Farben, und/oder unterschiedliche Hauptausrichtungen im Bezugssystem des ersten Bandes (4) aufweisen, wobei die Markierungen (9) periodisch im ersten eindimensionalen Markierungs-Netzwerk (11) und/oder im zweiten eindimensionalen Markierungs-Netzwerk (13) angeordnet sind.

6. System (1) nach einem der vorhergehenden Ansprüche, wobei der Hauptkörper (2) Mikronadeln umfasst, die zum Messen einer physiologischen Größe des Benutzers ausgelegt sind.

## Claims

1. Adjustable body monitoring system (1) comprising:
- a main body (2) adapted to measure a physiological quantity of a user,
- a wristband (3) configured to surround a limb of the user, the strap comprising a first strap (4) mounted on the main body (2) and a second strap (5) mounted on the main body (2), the first strap (4) comprising a one-dimensional array of notches (10), the one-dimensional array of notches (10) having a pitch *p,* the second strap (5) comprising fastening means (7) configured to be removably secured in one of the notches (6), each notch forming an opening through the first strap, wherein
- a first face (8) of the first strap (4) comprises a plurality of markers (9), each marker (9) being formed by a relief or a variation in color between the surface of the marker (9) and the rest of the first face (8), a portion of each marker (9) being arranged at a distance less than or equal to the pitch *p* of a notch (6) associated with said marker (9), preferably at a distance less than half the pitch *p* of the associated notch (6), so as to form a first one-dimensional array (11) of markers, two adjacent markers (9) of the first one-dimensional array (11) of markers having different shapes and/or different colors reflected by the first face (8), and/or different main orientations in the spatial reference frame of the first strap (4),
- a second face (12) of the first strap (4) opposite the first face (8), comprising a plurality of markers (9), each marker (9) being formed by a relief or a variation in color between the surface of the marker (9) and the rest of the second face (8), a portion of each marker (9) being arranged at a distance less than or equal to the pitch *p* of a notch (6) associated with said marker (9), preferably at a distance less than half the pitch *p* of the associated notch (6), so as to form a second one-dimensional array (13) of markers, two adjacent markers (9) of the second one-dimensional array (13) of markers having different shapes and/or different colors reflected by the second face (8), and/or different main orientations in the spatial reference frame of the first strap (4).

2. System according to claim 1, wherein a notch (6) is associated with an associated marker (9) on the first face and with an associated marker (9) on the second face, said associated markers having identical shapes and/or colors reflected by the first face (8) and by the second face (12) and/or identical main orientations in the reference frame of the first strap (4).

3. System according to one of the preceding claims, wherein the first and second networks of markers are identical, each marker is attached to the notch with which it is associated, each marker associated with each consecutive notch being different.

4. System according to one of claims 1 to 2, wherein the first and second marker networks are identical, the marker is constituted by a shape given to the notch such that at least each consecutive notch has a different shape.

5. System (1) according to one of the preceding claims, wherein the first one-dimensional array (11) of markers and/or the second one-dimensional array (13) of markers has at least two markers (9) having different shapes and/or different colors reflected by the first face (8) and/or by the second face (12), and/or different main orientations in the reference frame of the first strap (4), the markers (9) being arranged periodically in the first one-dimensional array (11) of markers and/or the second one-dimensional array (13).

6. System (1) according to one of the preceding claims, wherein the main body (2) comprises micro-needles configured to measure a physiological quantity of the user.
